Europäisches Patentamt

European Patent Office

Office européen des brevets

(11) Publication number: 0 135 045 B1

# EUROPEAN PATENT SPECIFICATION

(45) Date of publication of patent specification: 19.12.90

(21) Application number: 84108599.6

(22) Date of filing: 20.07.84

(51) Int. Cl.⁵: C 12 N 15/75, C 12 P 21/02

(54) A novel plasmid and methods for its use.

(30) Priority: 22.07.83 JP 135032/83

(43) Date of publication of application:
27.03.85 Bulletin 85/13

(45) Publication of the grant of the patent:
19.12.90 Bulletin 90/51

(84) Designated Contracting States:
BE DE FR GB NL SE

(56) References cited:
AGRICULTURAL AND BIOLOGICAL
CHEMISTRY, vol. 47, no. 5, May 1983, pages
1143-1149, Tokyo, JP; T. HARA et al.:
"Restriction endonuclease mapping of pUH1 in
Bacillus subtilis (natto)"
IDEM

AGRICULTURAL AND BIOLOGICAL
CHEMISTRY, vol. 47, no. 1, January 1983, pages
159-161; Y. TAKEICHI et al.: "Cloning of Bacillus
subtilis alpha-amylase structural gene in
plasmid pUB110"

(73) Proprietor: THE GREEN CROSS CORPORATION
3-3, Imabashi 1-chome Chuo-ku
Osaka-shi Osaka (JP)

(72) Inventor: Haruhide, Kawabe
3-29-14, Yamadanishi
Suita-shi, Osaka (JP)
Inventor: Hiromichi, Mukai
8-40-20, Matsugahana-cho Tennoji-ku
Osaka-shi, Osaka (JP)
Inventor: Hirofumi, Arimura
2-18-1-401, Uenosaka
Toyonaka-shi Osaka (JP)
Inventor: Tadakazu, Suyama
4-3-7, Matsuigaoka, Tanabe-cho
Tsuzuki-gun, Kyoto (JP)

(74) Representative: Hansen, Bernd, Dr.rer.nat. et al
Hoffmann, Eitle & Partner Patentanwälte
Arabellastrasse 4 Postfach 81 04 20
D-8000 München 81 (DE)

Courier Press, Leamington Spa, England.

**EP 0 135 045 B1**

56 References cited:

**MOLECULAR AND GENERAL GENETICS, vol. 181, 1981, pages 248-253, Springer-Verlag, DE; P. TICHY et al.: "Isolation of the pMI 10 plasmid from the alpha-amylase producing strain of Bacillus subtilis"**

**JOURNAL OF BACTERIOLOGY, vol. 131, no. 2, August 1977, pages 699-701, American Society for Microbiology, US; T. TANAKA et al.: "Isolation and characterization of four types of plasmids from Bacillus subtilis (natto)"**

**Description**

The present invention relates to a circular plasmid comprising at least an alpha-amylase promoter derived from *Bacillus subtilis (natto)*.

"Natto" produced by *B. subtilis (natto)* and steamed soybean, is one of the most traditional fermentation foods in Japan.

Plasmids, which are extrachromosomal genes, have been widely used in recently developed genetic engineering techniques as a vector for use *in vitro* recombination of genes. The *in vitro* genetic recombination technique has a wide application, e.g., in achieving useful microorganisms.

*Escherichia coil, B. subtilis,* yeast, etc. are mainly used as hosts in genetic engineering. However, when cloning of genes of higher organisms is attempted with the view to produce useful substances which higher organisms produce, such as growth hormones, etc., by means of microorganisms, yeast and *B. subtilis* may be used more advantageously than *E. coli*, since the former excrete their products outside the cells. A further advantage of the use of yeast and *B. subtilis* is that they are not harmful to humans nor parasitic to various animals and plants, thus providing a genetic operation system having a high safety.

An object of the present invention is to provide a plasmid vector permitting an efficient manifestation of genes introduced therein in host cells.

Another object of the present invention is to provide a plasmid vector of a high copy number.

Still another object of the present invention is to provide a plasmid vector which permits efficient production of various proteins in *B. subtilis* and *B. subtilis (natto)*.

Yet another object of the present invention is to provide a plasmid vector which can grow in both gram-positive and gram-negative bacteria.

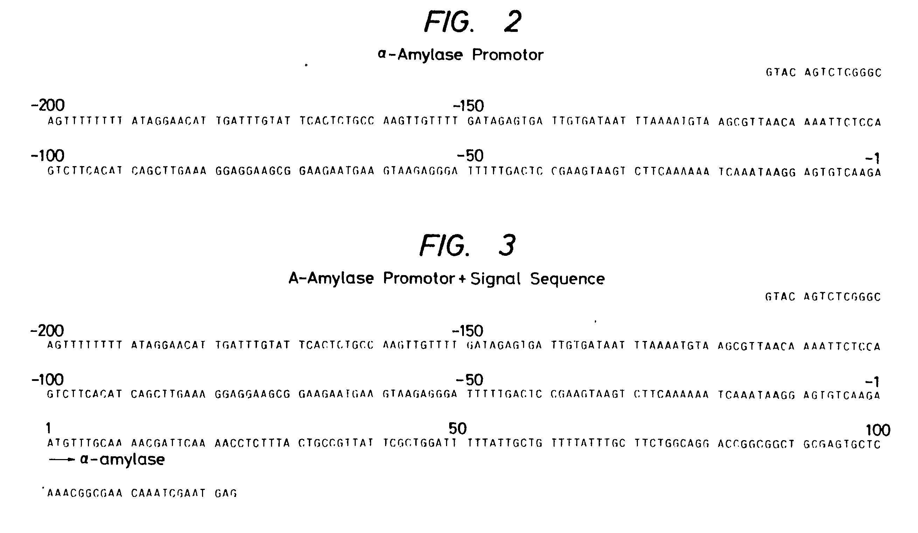

Extensive researches have been made in order to develop a new genetic operation system using *B. subtilis* and as a result thereof it has now been found that a new plasmid comprising at least an alpha-amylase promoter derived from *Bacillus subtilis (natto)*, wherein the alpha-amylase promoter has the base sequence shown in Fig. 2, is advantageous.

The present invention is based on the above finding.

Fig. 1 shows the base sequence of a fragment of a chromosomal DNA of *B. subtilis (natto)* coding for α-amylase and including an α-amylase promoter, a signal sequence and a part of structural gene.

Fig. 2 shows the base sequence of the α-amylase promoter in the DNA fragment shown in Fig. 1.

Fig. 3 shows the base sequence of the α-amylase promoter and the signal sequence in the DNA fragment shown in Fig. 1.

Fig. 4 schematically illustrates cloning of an α-amylase gene from chromosomal DNA of *B. subtilis (natto)*.

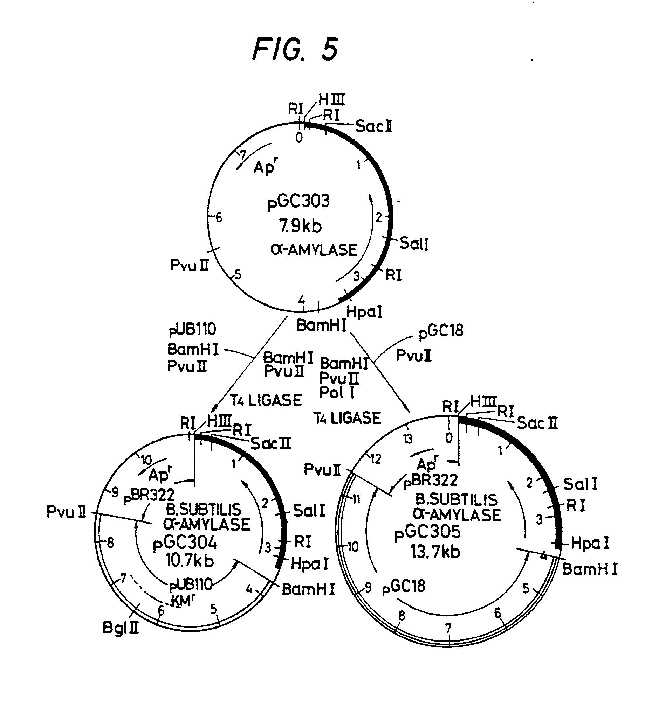

Fig. 5 schematically illustrates production of a shuttle vector containing an α-amylase gene.

In the drawings, the following abbreviations are used.

RI: *Eco* RI

H III: *Hind* III

SM$^r$: Streptomycin resistance

CM$^r$: Chloramphenicol resistance

KM$^r$: Kanamycin resistance

AP$^r$: Ampicillin resistance.

The alpha-amylase promoter gene may be obtained from *B. subtilis (natto)* GC 161, which has bacterial characteristics similar to those of *B. subtilis*. This strain which is particularly preferred as a source of an alpha-amylase promoter gene has been deposited at Fermentation Research Institute, Agency of Industrial Science and Technology, Ministry of International Trade and Industry, on June 6, 1984 under deposition No. FERM-BP No. 539.

The alpha-amylase promoter gene can be isolated from the chromosomal DNA of GC 161 strain in a conventional method as described in, for example, Palva, I.: *Gene 15*. 43—51 (1981).

The DNA thus obtained after having been digested into fragments with various known restriction enzymes, can be ligated with a known plasmid as an intermediate, and α-amylase gene-containing plasmids can be screened using starch-iodine reaction.

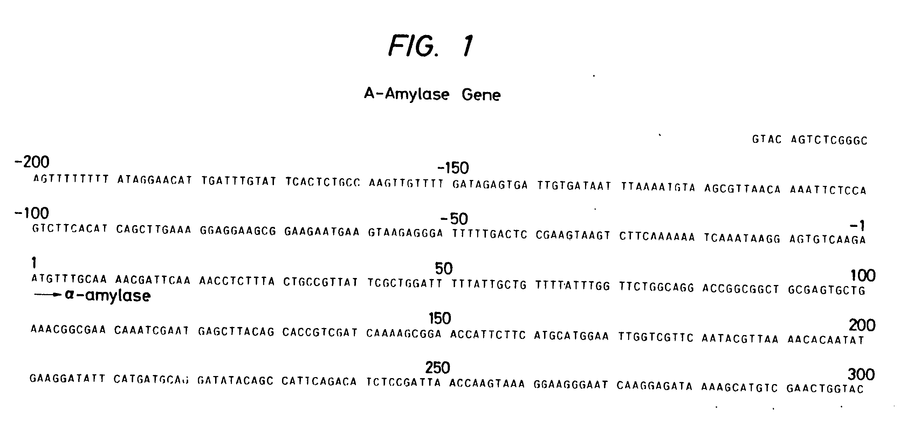

The base sequence of α-amylase gene DNA can be determined according to the method described by Maxam, A. and Gilbert, W.: *Methods in Enzymology 65,* 499—560 to obtain the base sequence illustrated in Fig. 1.

In the base sequence shown in Fig. 1, at most region −214 to −1 can be used as a promoter. If desired, smaller region beginning with −200 can be used (Fig. 2).

Further, promoter fragments can include a signal sequence i.e., base sequence of at most region 1 to 123 in addition to region −214 (or −200) to −1 (Fig. 3), if desired.

The α-amylase promoter containing plasmid of the present invention can be ligated with a plasmid vector derived from gram-negative bacteria such as *E. coli* (e.g., pBR 322) and further introduced in a plasmid vector derived from gram-positive bacterial as *B. subtilis, B. subtilis (natto), Staphylococcus aureus*, etc. (e.g., pUB 110) to obtain a shuttle vector containing an α-amylase promoter derived from *B. subtilis (natto)* and capable of growing in both gram-positive bacteria such as *B. subtilis, B. subtilis (natto)*, etc. and gram-negative bacteria such as *E. coli*.

Various genes coding for useful physiologically active substances can be inserted into a position downstream of the α-amylase promoter and signal sequence, and the resulting plasmid vectors can be used in transforming *B. subtilis, B. subtilis (natto), E. coli,* etc. preferably *B. subtilis,* or *B. subtilis (natto),* and causing the transformed bacteria to express newly introduced genetic information, thus efficiently producing desired proteins such as various interferons, urokinase, HB vaccine, etc.

Digestion of the extrachromosonal and chromosonal DNA or ligation of fragments of such DNA can be performed by mixing a DNA solution having a final DNA concentration of 0.5 to 10 microgram/microliter, preferably 1 microgram/microliter, a suitable buffer solution and a restriction enzyme or a ligase in an amount of 1 to 10, preferably 3, units/microgram DNA, and allowing the mixture to stand generally at 10 to 40°C, preferably 37°C for 2 hours to overnight, preferably 3 to 4 hours. In cases where *Bcl* I, *Sma* I and *Tag* T are used ranges of reaction temperature used are 20 to 55°C (preferably 50°C), 5 to 35°C (preferably 30°C) and 30 to 70°C (preferably 65°C), respectively. For partial digestion with *Rsa* I, 0.3 to 2, preferably 0.5 units/ microgram DNA of the enzyme is used and the reaction is continued for 5 minutes to 1 hour, preferably 10 minutes. When $T_4$ ligase is used the reaction is carried out at 0 to 37°C, preferably 16°C, for 2 hours to overnight preferably 16 hours.

Usually, the following buffer solutions are used for the digestion and ligation.

| Restriction Enzyme | Buffer Solution |
|---|---|
| *Eco* RI | 50 mM Tris-HCl, pH 7.5, 7 mM MgCl$_2$, 100 mM NaCl, 7 mM 2-Mercaptoethanol, 0.01% BSA |
| *Bam* HI | 10 mM Tris-HCl, pH 8.0, 7 mM MgCl$_2$, 100 mM NaCl, 2 mM 2-Mercaptoethanol, 0.01% BSA |
| *Hind* III | 10 mM Tris-HCl, pH 7.5, 7 mM MgCl$_2$, 60 mM NaCl |
| *Bgl* II | 10 mM Tris-HCl, pH 7.5, 7 mM MgCl$_2$, 100 mM NaCl, 7 mM 2-Mercaptoethanol |
| *Pst* I | 20 mM Tris-HCl, pH 7.5, 10 mM MgCl$_2$, 50 mM (NH$_4$)$_2$SO$_4$, 0.01% BSA |
| *Kpn* I | 6 mM Tris-HCl, pH 7.5, 6 mM MgCl$_2$, 5 mM 2-Mercaptoethanol, 0.02% BSA |
| *Pvu* II | 10 mM Tris-HCl, pH 7.5, 7 mM MgCl$_2$, 60 mM NaCl, 7 mM 2-Mercaptoethanol |
| *Hpa* I | 10 mM Tris-HCl, pH 7.5, 7 mM MgCl$_2$, 100 mM KCl, 7 mM 2-Mercaptoethanol, 0.01% BSA |
| *Hae* III | 10 mM Tris-Hcl, pH 7.5, 7 mM MgCl$_2$, 60 mM NaCl, 7 mM 2-Mercaptoethanol |
| *Hpa* II | 20 mM Tris-HCl, pH 7.4, 10 mM MgCl$_2$, 1 mM Dithiothreitol |
| *Xba* I | 10 mM Tris-HCl, pH 7.5, 7 mM MgCl$_2$, 100 mM NaCl, 7 mM 2-Mercaptoethanol, 0.01% BSA |
| *Bcl* I | 50 mM Tris-HCl, pH 8.0, 10 mM MgCl$_2$, 50 mM NaCl |
| *Sau* 96I | 10 mM Tris-HCl, pH 9.0, 12 mM MgCl$_2$, 100 mM KCl, 5 mM 2-Mercaptoethanol |
| *Cla* I | 10 mM Tris-HCl, pH 8.0, 10 mM MgCl$_2$, 100 microgram/ml BSA |
| *Ava* II | 10 mM Tris-HCl, pH 8.0, 7 mM MgCl$_2$, 60 mM NaCl, 7 mM 2-Mercaptoethanol |
| *Taq* I | 50 mM Tris-HCl, pH 8.0, 10 mM MgCl$_2$, 50 mM NaCl |
| *Sma* I | 10 mM Tris-HCl, pH 8.0, 7 mM MgCl$_2$, 20 mM KCl, 7 mM 2-Mercaptoethanol, 0.01% BSA |
| *Bal* I | 10 mM Tris-HCl, pH 8.0, 5 mM MgCl$_2$, 5 mM KCl, 0.1 mM ammonium sulfate, 1 mM 2-Mercaptoethanol, 100 microgram/ml BSA |
| *Rsa* I | 6 mM Tris-HCl, pH 8.0, 12 mM MgCl$_2$, 50 mM NaCl, 6 mM 2-Mercaptoethanol, 100 microgram/ml BSA |

| Restriction Enzyme | Buffer Solution |
|---|---|
| Eco RV | 10 mM Tris-HCl, pH 7.5, 7 mM MgCl$_2$, 150 mM NaCl, 7 mM 2-Mercaptoethanol, 0.01% BSA |
| Sac II | 6 mM Tris-HCl, pH 7.5, 6 mM MgCl$_2$, 6 mM 2-Mercaptoethanol, 100 microgram/ml BSA |
| DNA Polymerase I | 67 mM Potassium Phosphate, pH 7.4, 67 mM MgCl$_2$, 1 mM 2-Mercaptoethanol, 20 mM d (A-T) Copolymer, 33 microM dATP, 33 microM ($^3$H)-dTTP |
| T$_4$ DNA Ligase | 66 mM Tris-HCl, pH 7.6, 66 mM MgCl$_2$, 10 mM DTT, 0.1—1.0 mM ATP |

Experiment I
(Analysis of Base Sequence of α-amylase DNA from B. subtilis (natto)).

In order to know exact position of the α-amylase gene of B. subtilis (natto) GC161 in the DNA fragment obtained in Example 1 hereinbelow, the base sequence of the DNA fragment was determined according to the method described by Maxam, A. and Gilbert, W. (supra).

The base sequence of the promoter and structural gene of α-amylase DNA is shown in Fig. 1.

The present invention will be described in greater detail with reference to the following examples. However, it should not be construed as being limited thereto.

In Examples 1 and 2 below, digestion of DNA and ligation of DNA fragments were performed under the preferred conditions described hereinabove.

Example 1
Isolation of alpha-Amylase Gene

α-Amylase gene was isolated from the chromosomal DNA of B. subtilis (natto) GC161 described above. The isolation of the chromosomal DNA was performed according to a conventional method (Palva, I.: Gene 15, 43—51 (1981)) as follows.

B. Subtilis (natto) GC 161 strain was cultured overnight in 500 ml of L-broth (10 g of peptone, 5 g of yeast extract, 1 g of glucose and 5 g of NaCl, water to make 1 liter) at 30°C with shaking. And 1 liter of culture broth was centrifuged and washed to collect cells. To the cells were added 10 ml of a 20% sucrose-containing 5 mM Tris hydrochloride buffer solution (pH 8.0) and 100 mg of lysozyme, and the mixture was allowed to stand at 37°C for 15 minutes. Then, 10 ml of 1% sarcosyl — 1% EDTA solution and 100 mg/ml of pronase E were added to the reaction mixture, which was then allowed to stand at 37°C for 16 hours. The resulting solution was diluted with the TES solution to 35 ml followed by the addition of 35 g of CsCl and 1 ml of ethidium bromide solution (10 mg/ml), and centrifugation at 60,000 rpm for 16 hours using a Beckmann 70.1 Type Ti rotor.

Chromosomal DNA fraction was collected, which was removed of ethidium bromide with isoamyl alcohol in a conventional manner and dialyzed against TES solution followed by treatment with phenol twice to obtain purified chromosomal DNA.

The chromosomal DNA thus obtained was digested with restriction enzymes Pvu II, Bgl II, Hpa II, Bam HI, Hind III and subjected to electrophoresis on 0.8% agarose gel. DNA fragments were extracted from the agarose gel and ligated with various known plasmids (pC 194, pE 194, pUB 110, and pGC 200 derived from pC 194) and transformed to B. subtilis strain (rec⁻, amy⁻). The transformation was performed to spread on the L-agar plates containing both 1% soluble starch and an antibiotic, and incubated at 37°C for 2 days. Iodine solution (0.1% iodine — 1% KI) was dropped onto the agar plate and decoloration of iodine which indicates presence of α-amylase activity was checked. Of the transformed strains, a strain having plasmid pGC 301 (15 kb), which was obtained by inserting a DNA fragment of B. subtilis (natto) GC 161 digested with Hind III to pGC 200 (8 kb) at its Hind III position, showed α-amylase activity.

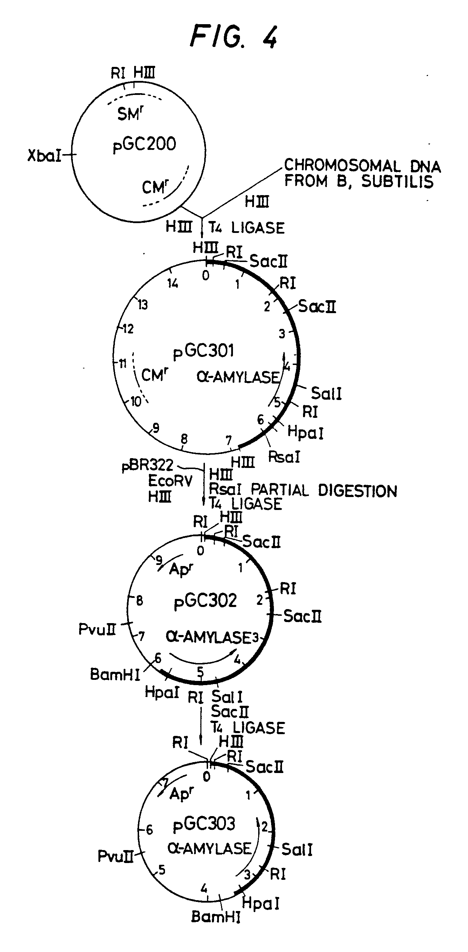

The pGC 301 was digested with Hind III completely and with Rsa I partially, and loaded on 0.8% agarose gel. The DNA fragments of various sizes obtained from agarose gel with ligated with a Eco RV-Hind fragment of pBR 322. Thus, pGC 302 (10 Kb) was obtained. Moreover, pGC 302 was digested with Suc II and a 7.9 Kb DNA fragment was isolated and ligated. Thus, pGC 303 was prepared (Fig. 4).

It was confirmed that the plasmids thus obtained contained the entire α-amylase gene. Thus, pGC 303 (7.9 kb) containing promotor, signal sequence and structural gene for α-amylase derived from B. subtilis (natto) pGC 161 was prepared (Fig. 1). pGC 303 is a plasmid whose host is E. coli.

Example 2

The pGC 303 obtained in Example 1 was ligated with pGC 18 as obtained in the following Example 3 and deposited at Fermentation Research Institute, Agency of Industrial Science and Technology, Ministry

5

of International Trade and Industry, on July 6, 1983 under deposition No. FERM-BP No. 312 (named *B. subtilis (natto)* GC 18), or pUB 110, derived from *Staphylococcus aureus* in a conventional manner.

pGC 303 was digested with *Bam* HI and *Pvu* II and pUB 110 was also digested with the same restriction enzymes. DNA fragments obtained were ligated with each other using $T_4$ ligase to prepare a hybrid plasmid pGC 304 having a molecular weight of 10.7 kb.

On the other hand, pGC 303 was digested with *Bam* HI, *Pvu* III and *Pol* I while pGC 18 was digested with *Pvu* II, and DNA fragments obtained were ligated with each other to give a hybrid plasmid pGC 305 having a molecular weight of 13.7 kb.

Example 3

*B. subtilis (natto)* GC 18 strain was cultured in 500 ml of L broth (10 g of peptone, 5 g of yeast extract, 1 g of glucose and 5 g of NaCl, water to make 1 liter) at 30°C with shaking, and cells were harvested by centrifugation. The cells thus collected were suspended in 18 ml of 25% sucrose TES solution (TES solution: 30 mM Tris HCl (pH 8.0), 50 mM, NaCl, and 5 mM EDTA).

To the cell suspension were added 1 ml of 0.5 M EDTA (pH 8.0) and 1 ml of lysozyme (10 mg/ml), and the mixture was allowed to react at 37°C for 30 minutes.

To the reaction mixture were added 10% SDS in 2 ml of TES and 2 ml of 5 M NaCl and the mixture was allowed to stand overnight at 4°C and then subjected to centrifugation (30,000 rpm) at 4°C for 30 minutes.

Polyethylene glycol was added to the supernatant to a final concentration of 10% and the mixture was allowed to stand overnight at 4°C, and subjected to centrifugation at 10,000 rpm for 10 minutes.

The residue was dissolved in TES and subjected to cesium chloride-ethidium bromide density gradient centrifugation at 60,000 rpm for 16 hours using a Beckmann Type 70.1 Ti rotor to obtain a plasmid DNA.

Then, the plasmid DNA was washed with isoamy alcohol in a conventional manner to remove ethidium bromide and dialyzed against 1,000 volumes of TES at 4°C.

## Claims

1. A circular plasmid comprising at least an alpha-amylase promoter derived from *Bacillus substilis (natto)*, wherein the alpha-amylase promoter has the base sequence shown in Fig. 2.

2. The circular plasmid as claimed in claim 1, wherein the plasmid comprises an alpha-amylase promoter and a signal sequence which are derived from *Bacillus subtilis (natto)* and have the base sequence shown in Fig. 3.

3. The circular plasmid as claimed in claim 1, wherein the plasmid further comprises a plasmid DNA base sequence derived from *E. coli.*

4. The circular plasmid as claimed in claim 3, wherein the plasmid DNA base sequence is a partial base sequence of pBR 322.

5. The circular plasmid as claimed in claim 3, wherein the plasmid further comprises a plasmid DNA base sequence derived from *Bacillus subtilis (natto)* or *Staphylococcus aureus.*

6. The use of a plasmid according to claims 1 to 5 for the production of peptides and proteins.

## Patentansprüche

1. Ringförmiges Plasmid, welches mindestens einen von *Bacillus subtilis (natto)* stammenden alpha-Amylase-Promotor umfasst, wobei der alpha-Amylase-Promotor die in Fig. 2 aufgeführte Basensequenz aufweist.

2. Ringförmiges Plasmid nach Anspruch 1, wobei das Plasmid einen alpha-Amylase-Promotor und eine Signalsequenz, die von *Bacillus subtilis (natto)* stammen, umfasst und die in Fig. 3 dargestellte Basensequenz aufweist.

3. Ringförmiges Plasmid nach Anspruch 1, wobei das Plasmid im weiteren eine Plasmid-DNA-Basensequenz, die von *E. coli* stammt, umfasst.

4. Ringförmiges Plasmid nach Anspruch 3, wobei die Plasmid-DNA-Basensequenz eine partielle Basensequenz von pBR 322 darstellt.

5. Ringförmiges Plasmid nach Anspruch 3, wobei das Plasmid im weiteren eine Plasmid-DNA-Basensequenz, die von *Bacillus subtilis (natto)* oder *Staphylococcus aureus* stammt, umfasst.

6. Verwendung eines Plasmids nach den Ansprüchen 1 bis 5 zur Herstellung von Peptiden und Proteinen.

## Revendications

1. Un plasmide circulaire comprenant au moins un promoteur d'α-amylase dérivé du *Bacillus subtilis (natto)*, selon lequel le promoteur d'α-amylase a la séquence de bases montrée à la figure 2.

2. Le plasmide circulaire selon la revendication 1, selon lequel le plasmide comprend un promoteur d'α-amylase et une séquence singal qui sont dérivés du *Bacillus subtilis (natto)* et qui ont la séquence de bases montrée à la figure 3.

3. Le plasmide circulaire selon la revendication 1, selon lequel le plasmide comprend en outre une séquence de bases d'ADN de plasmide dérivée du *E. coli.*

4. Le plasmide circulaire selon la revendication 3, selon lequel la séquence de bases de l'ADN de plasmide est une séquence de bases partielle du pBR 322.

5. Le plasmide circulaire selon la revendication 3, selon lequel le plasmide comprend en outre une séquence de bases d'ADN de plasmide dérivée du *Bacillus subtilis (natto)* ou du *Staphylococcus aureus.*

6. L'utilisation d'un plasmide selon l'une des revendications 1 à 5 pour la production des peptides et des protéines.

## FIG. 1

### A-Amylase Gene

EP 0 135 045 B1

```
                                                                                        GTAC AGTCTCGGGC
-200                                               -150
  AGTTTTTTTT ATAGGAACAT TGATTTGTAT TCACTCTGCC AAGTTGTTTT GATAGAGTGA TTGTGATAAT TTAAAATGTA AGCGTTAACA AAATTCTCCA

-100                                               -50
  GTCTTCACAT CAGCTTGAAA GGAGGAAGCG GAAGAATGAA GTAAGAGGGA TTTTTGACTC CGAAGTAAGT CTTCAAAAAA TCAAATAAGG AGTGTCAAGA
                                                                                                            -1

1                                                  50
  ATGTTTGCAA AACGATTCAA AACCTCTTTA CTGCCGTTAT TCGCTGGATT TTTATTGCTG TTTTATTTGG TTCTGGCAGG ACCGGCGGCT GCGAGTGCTG
  ──▶α-amylase                                                                                            100

                                                   150
  AAACGGCGAA CAAATCGAAT GAGCTTACAG CACCGTCGAT CAAAAGCGGA ACCATTCTTC ATGCATGGAA TTGGTCGTTC AATACGTTAA AACACAATAT
                                                                                                         200

                                                   250
  GAAGGATATT CATGATGCAG GATATACAGC CATTCAGACA TCTCCGATTA ACCAAGTAAA GGAAGGGAAT CAAGGAGATA AAAGCATGTC GAACTGGTAC
                                                                                                         300
```

# FIG. 2

## α-Amylase Promotor

GTAC AGTCTCGGGC

-200                                               -150

AGTTTTTTTT ATAGGAACAT TGATTTGTAT TCACTCTGCC AAGTTGTTTT GATAGAGTGA TTGTGATAAT TTAAAATGTA AGCGTTAACA AAATTCTCCA

-100                                               -50                                             -1

GTCTTCACAT CAGCTTGAAA GGAGGAAGCG GAAGAATGAA GTAAGAGGGA TTTTTGACTC CGAAGTAAGT CTTCAAAAAA TCAAATAAGG AGTGTCAAGA

# FIG. 3

## A-Amylase Promotor + Signal Sequence

GTAC AGTCTCGGGC

-200                                               -150

AGTTTTTTTT ATAGGAACAT TGATTTGTAT TCACTCTGCC AAGTTGTTTT GATAGAGTGA TTGTGATAAT TTAAAATGTA AGCGTTAACA AAATTCTCCA

-100                                               -50                                             -1

GTCTTCACAT CAGCTTGAAA GGAGGAAGCG GAAGAATGAA GTAAGAGGGA TTTTTGACTC CGAAGTAAGT CTTCAAAAAA TCAAATAAGG AGTGTCAAGA

1                                               50                                             100

ATGTTTGCAA AACGATTCAA AACCTCTTTA CTGCCGTTAT TCGCTGGATT TTTATTGCTG TTTTATTTGC TTCTGGCAGG ACCGGCGGCT GCGAGTGCTC

⟶ α-amylase

AAACGGCGAA CAAATCGAAT GAG

# FIG. 4

# FIG. 5